# EUROPEAN PATENT APPLICATION

(11) **EP 2 311 395 A1**
(43) Date of publication of application: **20.04.2011**
(21) Application number: 10251796.8
(22) Date of filing: 13.10.2010
(51) Int. Cl.: A61B 17/34

(54) **Flexible access device for use in surgical procedures**

(30) Priority: 14.10.2009 US 578832
(71) Applicant: Tyco Healthcare Group LP, New Haven, CT 06511 (US)
(72) Inventor: Racenet, Danyel J., Middletown, CT 06457 (US)
(74) Representative: Soames, Candida Jane

(57) **Abstract**

A flexible access device for insertion through tissue is provided. The flexible access device includes a compressible body having a first collapsed configuration and a second resiliently expanded configuration. The body is compressible in both a radial dimension and a longitudinal dimension and is resilient to expand in an incision in the tissue. The body includes a trailing end defining concave receiving recess and a leading end defining a concave exiting recess. The flexible access device further includes a lumen disposed in the body and extending therethrough, the lumen communicating with the concave receiving and exiting recesses so as to receive an instrument with a non-linear shaft.

## Description

### CROSS REFERENCE TO RELATED APPLICATION

This application is a continuation-in-part of U.S. Patent Application Serial No. 12/244,024, filed on October 2, 2008, which claims the benefit of and priority to U. S. Provisional Patent Application Serial No. 60/075,867, filed June 26, 2008, entitled SEAL ANCHOR FOR USE IN SURGICAL PROCEDURES, and U.S. Provisional Application Serial No. 60/997,885, filed on October 5, 2007, entitled SEAL ANCHOR FOR USE IN SINGLE INCISION SURGERY, the entire content of each application is incorporated herein by reference.

### BACKGROUND

### 1. Technical Field

The present disclosure relates to flexible access assemblies for use in surgical procedures. More particularly, the present disclosure relates to a flexible access device having one or more lumens or ports capable of receiving a surgical instrument with a straight, irregular or curved elongated shaft.

### 2. Background of the Related Art

Today, many surgical procedures are performed through small incisions in the skin, as compared to the larger incisions typically required in traditional procedures, in an effort to reduce both trauma to the patient and recovery time. Some of these procedures are referred to as "endoscopic", and if performed in the patient's abdomen, the procedure is referred to as "laparoscopic".

During a typical minimally invasive procedure, surgical objects, such as surgical access devices, e.g., trocar and cannula assemblies, endoscopes, or other instruments, are inserted into the patient's body through the incision in tissue. Prior to the introduction of the surgical object into the patient's body, insufflation gasses may be used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various access members are used during the course of minimally invasive procedures and are widely known in the art. However, a continuing need exists for an access member that can be inserted directly into the incision in tissue, that can support valves and seals or receive surgical instruments directly, and that can accommodate a variety of surgical objects while maintaining the integrity of an insufflated workspace. It is desirable to accommodate instruments with straight, curved or irregularly shaped shafts.

### SUMMARY

Accordingly, a flexible access device for insertion through tissue is provided. The flexible access device includes a compressible body having a first collapsed configuration and a second resiliently expanded configuration and a lumen disposed in the body and extending therethrough. The body is compressible in both a radial dimension and a longitudinal dimension and is resilient to expand in an incision in the tissue. The body includes a trailing end defining concave receiving recess and a leading end defining a concave exiting recess. The lumen communicates with the concave receiving and exiting recesses so as to receive an instrument with a non-linear shaft. The body may include a central portion and the trailing end may include a positioning member. The leading end of the body may include a positioning member. The positioning member may have a diameter greater than a diameter of the central portion. The body may include a coating that is at least one of parylene, hydrophilic, hydrophobic, bio-agents, anti-infection and analgesic.

Also provided is a method of accessing an abdominal cavity. The method includes the steps of creating an incision through the abdominal wall, providing a flexible access device having a body and a port extending through the body, the lumen for forming a seal with a non-linear instrument disposed in the port, compressing the body such that it may be inserted through the incision, inserting the compressed body through the incision, releasing the compressed body to permit the body to return towards an original shape and receiving a non-linear instrument through the port. The method may further include the step of removing the non-linear instrument. The body includes a Parylene coating.

A kit for performing a lower anterior resection is also provided. The kit includes a surgical instrument having a pair of jaws for applying surgical fasteners to tissue, the pair of jaws having free ends and a curved configuration and a flexible access device. The flexible access device includes a compressible body having a first collapsed configuration and a second resiliently expanded configurations, the body being compressible in both a radial and longitudinal dimensions and being resilient to expand in an incision in the tissue, the body having a trailing end and a leading end defining concave receiving and exiting recesses and a lumen disposed in the body and extending therethrough, the lumen communicating with the concave receiving and exiting recesses so as to receive an instrument with a non-linear shaft. The surgical instrument included in the kit may include a surgical stapling cartridge.

### BRIEF DESCRIPTION OF THE DRAWINGS

Various embodiments of the present disclosure are described hereinbelow with references to the drawings, wherein:

**FIG. 1** is a front perspective view of a surgical apparatus in accordance with the principles of the present disclosure shown in an expanded condition illustrating a seal anchor member positioned relative to the tissue;

**FIG. 2** is a cross-sectional view of the seal anchor member of **FIG. 1** taken along line **2-2** of **FIG. 1** illustrating a port that extends longitudinally therethrough;

**FIG. 3** is a view of the port of **FIG. 2** with a surgical object inserted therethrough;

**FIG. 4** is a perspective view of the seal anchor member of **FIG. 1** shown in a compressed condition and prior to the insertion thereof into an incision in tissue;

**FIG. 5** is a front perspective view of the seal anchor member shown in the expanded condition and subsequent to its insertion into the incision;

**FIG. 6** is an exploded perspective view of an exemplary cannula for insertion within the longitudinal extending port of the seal anchor member;

**FIG. 7** is a front perspective view of an alternate embodiment of the surgical apparatus of **FIG. 1** illustrating a seal anchor member and an inflatable fluid membrane;

**FIG. 7A** is a front perspective view of the fluid port of the fluid membrane;

**FIG. 7B** is a front perspective view of the fluid port of **FIG. 7A** with the valve in an open position; and

**FIG. 8** is a front perspective view of the seal anchor member of the surgical apparatus of in compressed condition prior to the insertion within the incision.

**FIG. 9** is a top perspective view of an alternate embodiment of the seal anchor member of **FIG. 1** having concave proximal and distal portions;

**FIG. 10** is a side view of the seal anchor member of **FIG. 9****;**

**FIG. 11** is a top view of the seal anchor member of **FIG. 9****;**

**FIG. 12** is a cross-sectional view of the seal anchor member of **FIG. 9** taken along line **12-12** of **FIG. 11** illustrating a port that extends longitudinally therethrough;

**FIG. 13** is a cross-sectional view of the seal anchor member of **FIG. 9** taken along line **13-13** of **FIG. 10****;**

**FIG. 14** is a front perspective view of another embodiment of the seal anchor member of **FIG. 1** having convex proximal and distal portions;

**FIG. 15** is a top, perspective view of yet another embodiment of the seal anchor member of **FIG. 1** shown in an expanded condition with a surgical object inserted into one of the ports extending longitudinally therethrough;

**FIG. 16** is a perspective, cross-sectional view of the seal anchor member of **FIG. 15** taken along line **16-16;**

**FIG. 17** is a top, perspective view of still another embodiment of the seal anchor member of **FIG. 1** shown in an expanded condition with a surgical object inserted into one of the ports extending longitudinally therethrough;

**FIG. 18** is a perspective, cross-sectional view of the seal anchor member of **FIG. 17** taken along line **18-18;**

**FIG. 19** is a top view of an alternate embodiment of the seal anchor member seen in **FIG. 1** including an ingress port and an egress port each extending longitudinally therethrough;

**FIG. 20** is a side, cross-sectional view of the seal anchor member of **FIG. 19** positioned within a patient's tissue;

**FIG. 21** is a side, perspective view of a tube assembly for insertion into the ingress port of one embodiment of the seal anchor member of **FIG. 19****;**

**FIG. 22** illustrates a first kit in accordance with the principles of the present disclosure including the seal anchor member of **FIG.19** and a plurality of obturators positionable within a plurality of cannulae;

**FIG. 23** illustrates an alternate embodiment of the kit of **FIG. 22****;**

**FIG. 24** illustrates another alternate embodiment of the surgical kit including a seal anchor member and an insufflation/evacuation implement;

**FIG. 25** is a top plan view of the seal anchor member and the insufflation/evacuation implement of the surgical kit of **FIG. 24****;**

**FIG. 26** is a side cross-sectional view of the seal anchor member and the insufflation/evacuation implement taken along the lines **26-26** of **FIG. 25****;**

**FIG. 27** illustrates additional instrumentation incorporated within the surgical kit of **FIGS. 24-26****;**

**FIGS. 28A-28C** illustrate a method of use of the surgical kit of **FIGS. 24-27****;**

**FIG. 29** is a perspective view of a flexible access device of the present disclosure;

**FIG. 30** is a top view of the flexible access device of **FIG. 29****;**

**FIG. 31** is a cross-sectional side view of the flexible access device of **FIGS. 29** and 30;

**FIG. 32** is a perspective view of the flexible access device of **FIGS. 29-31** in a compressed condition and ready for insertion through the incision in the tissue;

**FIG. 33** is a perspective view of the flexible access device of **FIGS. 29-31** positioned through the incision in the tissue;

**FIG. 34** is a cross-sectional side view of the flexible access device of **FIGS. 29**-**31,** including a instrument having a curved portion being inserted therethrough;

**FIG. 35** is a cross-sectional side view of the flexible access device of **FIGS. 29**-**31,** including a instrument having a curved portion having been inserted therethrough; and

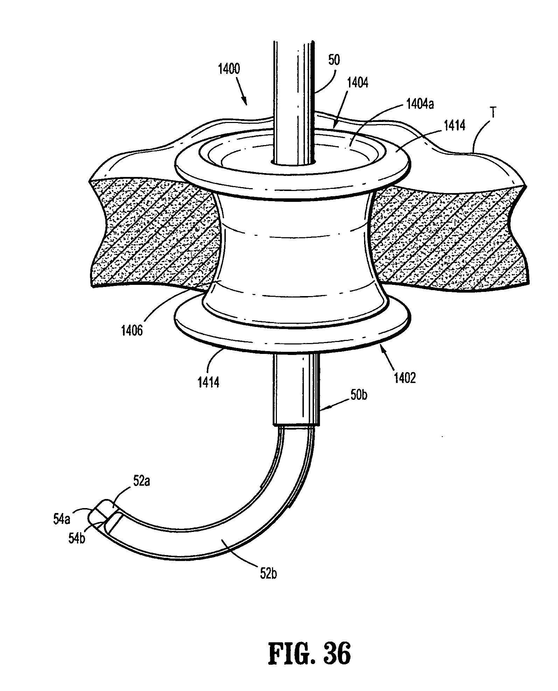

**FIG. 36** is partial cross-sectional side view of the flexible access device of **FIGS. 29-31** received through tissue and including an instrument having a curved shaft inserted therethrough.

### DETAILED DESCRIPTION OF THE EMBODIMENTS

In the drawings and in the description which follows, in which like references numerals identify similar or identical elements, the term "proximal" will refer to the end of the apparatus which is closest to the clinician during use, while the term "distal" will refer to the end which is furthest from the clinician, as is traditional and known in the art.

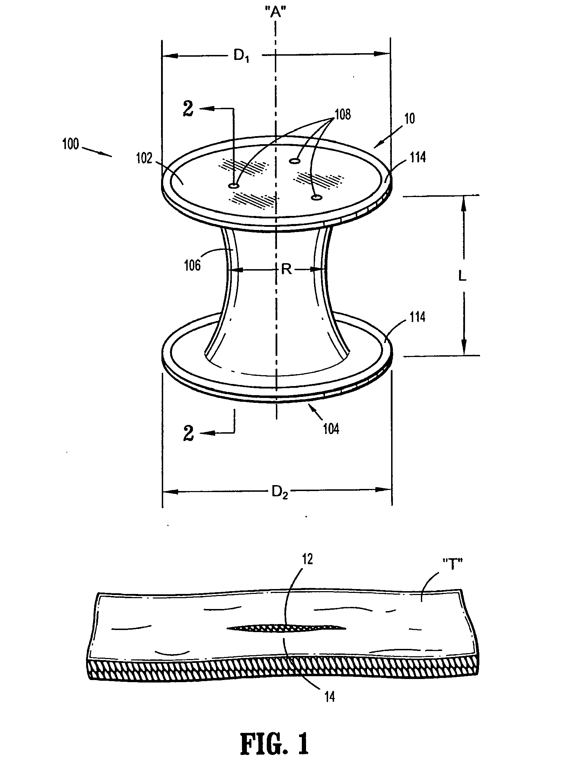

With reference to **FIGS. 1-3****,** a surgical apparatus **10** for use in a surgical procedure, e.g., a minimally invasive procedure is illustrated. Surgical apparatus **10** includes seal anchor member **100** defining a longitudinal axis "**A**" and having respective trailing (or proximal) and leading (or distal) ends **102, 104** and an intermediate portion **106** disposed between the trailing and leading ends **102, 104.** Seal anchor member **100** includes one or more ports **108** that extend longitudinally between trailing and leading ends **102, 104,** respectively, and through seal anchor member **100.**

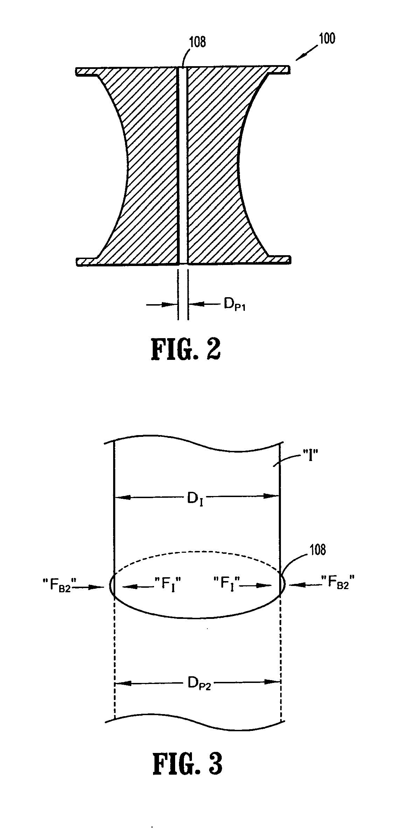

Seal anchor member **100** is preferably formed from a suitable foam material having sufficient compliance to form a seal about one or more surgical objects, shown generally as surgical object "**I**" (**FIG. 3**), and also establish a sealing relation with the tissue. The foam is preferably sufficiently compliant to accommodate off axis motion of the surgical object "**I**". In one embodiment, the foam includes a polyisoprene material.

Proximal end **102** of seal anchor member defines a first diameter **D₁** and distal end **104** defines a second diameter **D₂.** In one embodiment of seal anchor member **100,** the respective first and second diameters **D₁, D₂** of the proximal and distal ends **102, 104** are substantially equivalent, as seen in **FIG. 1****,** although an embodiment of seal anchor member **100** in which diameters **D_{1,} D₂** are different is also within the scope of the present disclosure. As depicted in **FIG. 1****,** proximal and distal ends **102, 104** define substantially planar surfaces. However, embodiments are also contemplated herein in which either or both of proximal and distal ends **102, 104,** respectively, define surfaces that are substantially arcuate to assist in the insertion of seal anchor member **100** within a tissue tract **12** defined by tissue surfaces **14** and formed in tissue **"T",** e.g., an incision, as discussed in further detail below.

Intermediate portion **106** defines a radial dimension **"R"** and extends longitudinally between proximal and distal ends **102, 104,** respectively, to define an axial dimension or length **"L".** The radial dimension **"R"** of intermediate portion **106** varies along the axial dimension, or length, **"L"** thereof. Accordingly, seal anchor member **100** defines a cross-sectional dimension that varies along its length **"L",** which facilitates the anchoring of seal anchor member **100** within tissue **"T",** as discussed in further detail below. However, an embodiment of seal anchor member **100** in which the radial dimension **"R"** remains substantially uniform along the axial dimension **"L"** thereof is also within the scope of the present disclosure.

The radial dimension **"R"** of intermediate portion **106** is appreciably less than the respective diameters **D₁, D₂** of proximal and distal ends **102, 104** such that seal anchor member **100** defines an "hour-glass" shape or configuration to assist in anchoring seal anchor member **100** within tissue **"T",** as discussed in further detail below. However, in an alternate embodiment, the radial dimension **"R"** of intermediate portion **106** may be substantially equivalent to the respective diameters **D₁, D₂** of proximal and distal ends **102, 104.** In cross section, intermediate portion **106** may exhibit any suitable configuration, e.g., substantially circular, oval or oblong.

Each port **108** is configured to removably receive the surgical object **"I".** Prior to the insertion of surgical object **"I",** port **108** is in a first state in which port **108** defines a first or initial dimension **D_{P1}. D_{P1}** will generally be about **0**mm such that the escape of insufflation gas (not shown) through port **108** of seal anchor member **100** in the absence of surgical object **"I"** is substantially prevented. For example, port **108** may be a slit extending the longitudinal length of seal anchor member **100** through proximal and distal ends **102, 104.** In the alternative, port **108** may define an opening within seal anchor member **100** having an initial open state. Upon the introduction of surgical object **"I",** port **108** transitions to a second state in which port **108** defines a second, larger dimension **D_{P2}** that substantially approximates the diameter **D₁** of surgical object **"I"** such that a substantially fluid-tight seal is formed therewith, thereby substantially preventing the escape of insufflation gas (not shown) through port **108** of seal anchor member **100** in the presence of surgical object **"I". D₁,** and thus **D_{P2},** will generally lie within the range of about **5**mm to about **12**mm, as these dimensions are typical of the surgical objects used during the course of minimally invasive procedures. However, a seal anchor member **100** including a port **108** that is capable of exhibiting substantially larger, or smaller, dimensions in the second state thereof is not beyond the scope of the present disclosure. In addition, seal anchor **100** may be devoid of ports **108.** With this arrangement, ports **108** are created within seal anchor member **100** during the insertion of the surgical object **"I".** In accordance with this embodiment, seal anchor member **100** is formed of a flowable or sufficiently compliable material such as a foam material, e.g., an open-cell polyurethane foam, a thermoplastic elastomer (TPE) or a gel. The formation of seal anchor member **100** may involve a process whereby an inert gas, such as CO2 or nitrogen is infused into the material so as to form a foam structure. Seal anchor member **100** may also be coated with lubricious coating, e.g., Parylene N or C in order to ease insertion of instruments and/or cannulas therethrough.

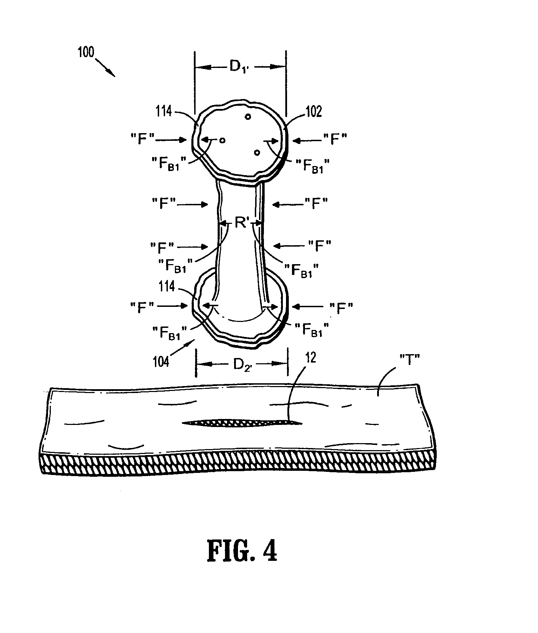

Referring now to **FIGS. 1** and **4****,** seal anchor member **100** is adapted to transition from an expanded condition (**FIG. 1**) to a compressed condition (**FIG. 4**) so as to facilitate the insertion and securement thereof within tissue tract **12** in tissue **"T".** In the expanded condition, seal anchor member **100** is at rest and the respective radial dimensions **D₁, D₂** of the proximal and distal ends **102, 104** of seal anchor member **100,** as well as the radial dimension **R** of the intermediate portion **106** are such that the seal anchor member **100** cannot be inserted within tissue tract **12.** However, as seen in **FIG. 4**, in the compressed condition, proximal and distal ends **102, 104** of seal anchor member **100,** as well as intermediate portion **106** are dimensioned for insertion into tissue tract **12.**

Seal anchor member **100** is formed of a biocompatible compressible material that facilitates the resilient, reciprocal transitioning of seal anchor member **100** between the expanded and compressed conditions thereof. In one embodiment, the compressible material is a "memory" foam. An external force **"F"** is applied to seal anchor member **100** to cause the seal anchor member **100** to assume the compressed condition. External force **"F"** is directed inwardly and when seal anchor member **100** is subjected thereto, e.g., when seal anchor member **100** is squeezed, seal anchor member **100** undergoes an appreciable measure of deformation, thereby transitioning into the compressed condition.

As depicted in **FIG. 4****,** as seal anchor member **100** is compressed under the influence of external force **"F",** an internal biasing force **"F_{B1}"** is created within seal anchor member **100** that is directed outwardly, opposing force **"F".** Internal biasing force **"F_{B1}"** endeavors to expand seal anchor member **100** and thereby return seal anchor member **100** to the expanded condition thereof. Accordingly, as long as seal anchor member **100** is subject to external force **"F",** seal anchor member **100** remains in the compressed condition. Upon the removal of external force **"F",** however, biasing force **"F_{B1}"** acts to return seal anchor member **100** to the expanded condition.

The compressible material comprising seal anchor member **100** also facilitates the resilient transitioning of port **108** between its first closed state (**FIGS. 1-2**) and its second state (**FIG. 3**). As previously discussed, prior to the insertion of surgical object "**I**", port **108** is in its first state in which port **108** defines a first or initial dimension **D_{P1}.** Port **108** may incorporate a slit extending the longitudinal length of seal anchor member **100.** In this first state, port **108** is at rest and is not subject to any external forces. However, upon the introduction of surgical object **"I"** through port **108** as depicted in **FIG. 3****,** the surgical object **"I"** exerts a force **"F₁"** upon port 108 that is directed radially outward. Force **"F₁"** acts to enlarge the dimensions of port **108** and thereby transition port **108** into the second state thereof in which port **108** defines a second, larger dimension **D_{P2}** that substantially approximates the diameter **D₁** of surgical object **"I".** Consequently, an internal biasing force **"F_{B2}"** is created that is directed radially inward, in opposition to force **"F₁".** Internal biasing force **"F_{B2}"** endeavors to return port **108** to reduce the internal dimension of port **108** and thereby return port **108** to the first state thereof. Internal biasing force **"F_{B2}"** is exerted upon surgical object **"I"** and acts to create a substantially fluid-tight seal therewith. The significance of forces **"F_{B1}"** and **"F_{B2}"** will be discussed in further detail below.

Referring again to **FIG. 1****,** one or more positioning members **114** may be associated with either or both of trailing (or proximal) end **102** and distal (or leading) end **104** of seal anchor member **100.** Positioning members **114** may be composed of any suitable biocompatible material that is at least semi-resilient such that positioning members **114** may be resiliently deformed and may exhibit any suitable configuration, e.g., substantially annular or oval. Prior to the insertion of seal anchor member **100,** positioning members **114** are deformed in conjunction with the respective proximal and distal ends **102, 104** of seal anchor member **100** to facilitate the advancement thereof through tissue tract **12** (**FIG. 4**). Subsequent to the insertion of seal anchor member **100** within tissue tract **12,** the resilient nature of positioning members **114** allows positioning members to return to their normal, substantially annular configuration, thereby aiding in the expansion of either or both of the respective proximal and distal ends **102, 104** and facilitating the transition of seal anchor member **100** from its compressed condition to its expanded condition. Positioning members **114** also may engage the walls defining the body cavity to further facilitate securement of seal anchor member **100** within the body tissue. For example, positioning member **114** at leading end **104** may engage the internal peritoneal wall and positioning member **114** adjacent trailing end **102** may engage the outer epidermal tissue adjacent the incision **12** within tissue **"T".** In another embodiment of seal anchor member **100,** one or more additional positioning members **114** may be associated with intermediate portion **106.**

The use and function of seal anchor member **100** will be discussed during the course of a typical minimally invasive procedure. Initially, the peritoneal cavity (not shown) is insufflated with a suitable biocompatible gas such as, e.g., CO₂ gas, such that the cavity wall is raised and lifted away from the internal organs and tissue housed therein, providing greater access thereto. The insufflation may be performed with an insufflation needle or similar device, as is conventional in the art. Either prior or subsequent to insufflation, a tissue tract **12** is created in tissue "T", the dimensions of which may be varied dependent upon the nature of the procedure.

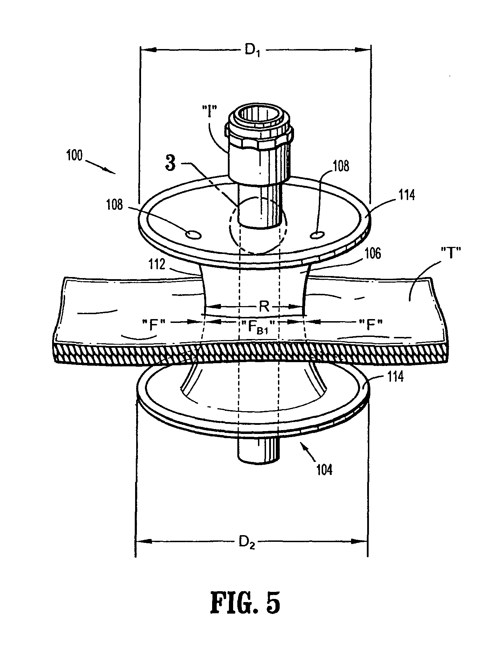

Prior to the insertion of seal anchor member **100** within tissue tract **12,** seal anchor member **100** is in its expanded condition in which the dimensions thereof prohibit the insertion of seal anchor member **100** into tissue tract **12.** To facilitate insertion, the clinician transitions seal anchor member **100** into the compressed condition by applying a force **"F"** thereto, e.g., by squeezing seal anchor member **100.** Force **"F"** acts to reduce the radial dimensions of the proximal and distal ends **102, 104,** respectively, to **D₁'** and **D₂'** (**FIG. 4**) including positioning members **114** (if provided) and to reduce the radial dimension of intermediate portion **106** to **R'** such that seal anchor member **100** may be inserted into tissue tract **12.** As best depicted in **FIG. 5****,** subsequent to its insertion, distal end **104,** positioning member **114** (if provided) and at least a section **112** of intermediate portion **106** are disposed beneath the tissue **"T".** Seal anchor member **100** is caused to transition from the compressed condition to the expanded condition by removing force **"F"** therefrom.

During the transition from the compressed condition to the expanded condition, the dimensions of seal anchor member **100,** i.e., the respective radial dimensions **D₁', D₂'** (**FIG. 4**) of the proximal and distal ends **102, 104** are increased to **D₁** and **D₂** (**FIG. 5**) and the radial dimension **R'** is increased to **R.** The expansion of distal end **104** is relatively uninhibited given the disposition thereof beneath tissue **"T",** and accordingly, distal end **104** is permitted to expand substantially, if not completely. However, as seen in **FIG. 5****,** the expansion of the section **112** of the intermediate portion **106** is limited by the tissue surfaces **14** (**FIG. 1**) defining tissue tract **12,** thereby subjecting intermediate portion **106** to an external force **"F"** that is directed inwardly. As discussed above, this creates an internal biasing force **"F_{B1}"** that is directed outwardly and
exerted upon tissue surfaces **14,** thereby creating a substantially fluid-tight seal between the seal anchor member **100** and tissue surfaces **14** and substantially preventing the escape of insufflation gas around seal anchor member **100** and through tissue tract **12.**

In the expanded condition, the respective radial dimensions **D₁, D₂** of the proximal and distal ends **102, 104** are substantially larger than the radial dimension **R** of the intermediate portion **106** thereby giving seal anchor member **100** the aforedescribed "hour-glass" configuration. Subsequent to insertion, the radial dimension **D₂** of distal end **104** and positioning member **114** is also substantially larger than the dimensions of the tissue tract **12.** Consequently, seal anchor member **100** may not be removed from tissue tract **12** in the expanded condition and thus, seal anchor member **100** will remain anchored within the tissue **"T"** until it is returned to its compressed condition.

After successfully anchoring seal anchor member **100** within the patient's tissue **"T",** one or more surgical objects **"I"** may be inserted through ports **108.** **FIG. 5** illustrates a surgical object **"I"** introduced through one of ports **108.** As previously discussed, prior to the insertion of surgical object **"I",** port **108** is in its first state in which port **108** defines an initial dimension **D_{P1}** which may be negligible in that port **108,** in one embodiment, is a longitudinal slit. Accordingly, prior to the escape of insufflation gas through port **108,** in the absence of surgical object **"I"** is minimal, thereby preserving the integrity of the insufflated workspace.

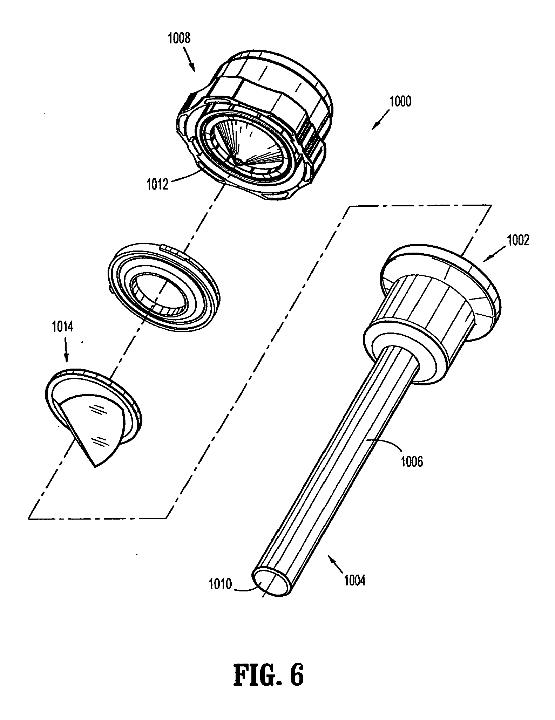

Surgical object **"I"** may be any suitable surgical instrument and, accordingly, may vary in size. Suitable surgical objects to be introduced within one or more of the ports **108** include minimally invasive grasper instruments, forceps, clip-appliers, staplers, etc. It is further contemplated that the surgical objects may include a conventional cannula **1000** as depicted in **FIG. 6****.** Cannula **1000** is configured for removable insertion into port **108** and includes respective proximal and distal ends **1002, 1004,** a shaft or elongate member **1006** disposed therebetween and seal housing **1008.** Elongate member **1006** defines an opening **1010** extending longitudinally therethrough that is dimensioned to permit the passage of surgical instrumentation (not shown), such as an obturator. Disposed within seal housing **1008** is an instrument seal **1012** that is adapted to receive the surgical instrumentation inserted into longitudinal opening **1010** so as to form a substantially fluid-tight seal therewith. Cannula **1000** further includes a closure valve **1014** that is biased into a closed position, but is adapted to open upon the introduction of the surgical instrumentation inserted into longitudinal opening **1010** to allow the surgical instrumentation to pass therethrough. In the closed position, i.e., in the absence of surgical instrumentation, closure valve **1014** prevents the communication of insufflation gas therethrough.

Upon the introduction of surgical object **"I",** e.g., cannula **1000,** port **108** is enlarged, thereby transitioning into its second state in which port **108** defines a second dimension **D_{P2}** (**FIG. 3**) that substantially approximates the diameter **D₁** of surgical object **"I",** thereby creating a substantially fluid tight seal with surgical object **"I"** and substantially preventing the escape of insufflation gas (not shown) through port **108** of seal anchor member **100** in the presence of a surgical object **"I",** as previously discussed.

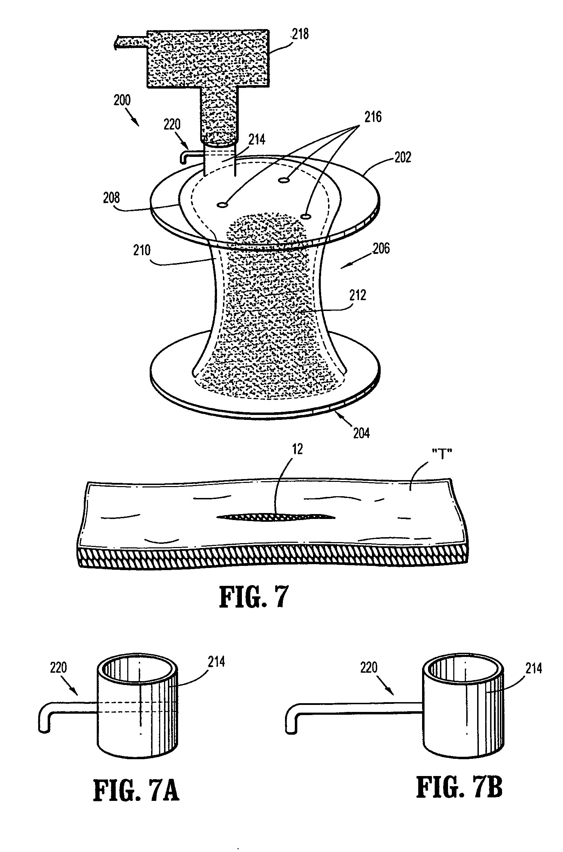

Referring now to **FIGS. 7-8****,** an alternate embodiment of a seal anchor member 200 is disclosed. Seal anchor member **200** comprises a resilient conformable material such as foam or, alternatively, a gel. Seal anchor member **200,** proximal and distal ends **202, 204,** and an intermediate portion **206** disposed therebetween. Seal anchor member **200** further includes expandable membrane **208** defining internal cavity **210.** Membrane **208** may be, e.g., substantially annular or donut-shaped in configuration, although any conceivable shape may be employed, and may be secured, attached or embedded to or within the foam or gel material of seal anchor member **200.** In one embodiment, membrane **208** surrounds foam or gel segment **212** thereby defining the periphery of seal anchor member **200.** One or more fluid ports **214** are in communication with internal cavity **210** of membrane **208** and one or more longitudinal ports **216** that extend through foam segment **212** of seal anchor member **200.**

Internal cavity **210** defined by membrane **208** is configured to retain a fluid therein. Membrane **208** may be formed of any suitable biocompatible that is sufficiently resilient to allow the flow of fluid into and out of internal cavity **210** to cause the expansion and contraction thereof. In addition, the material comprising membrane **208** is substantially impermeable with respect to the fluid to ensure that the flow of fluid into and out of internal cavity occurs solely through fluid port **214.**

Fluid port **214** is adapted for connection to a fluid source **218.** Fluid port **214** may be any member or structure suitable for this intended purpose. Although depicted as including a single fluid port **214,** in alternate embodiments, seal anchor member **200** may include additional fluid ports, e.g., on each of proximal and distal ends **202, 204,** respectively. Fluid port **214** may also include a valve **220** that is selectively positionable between an open position (**FIG. 7A**) and a closed position (**FIG. 7B**) to regulate the flow of fluid into and out of internal cavity **210** through fluid port **214.**

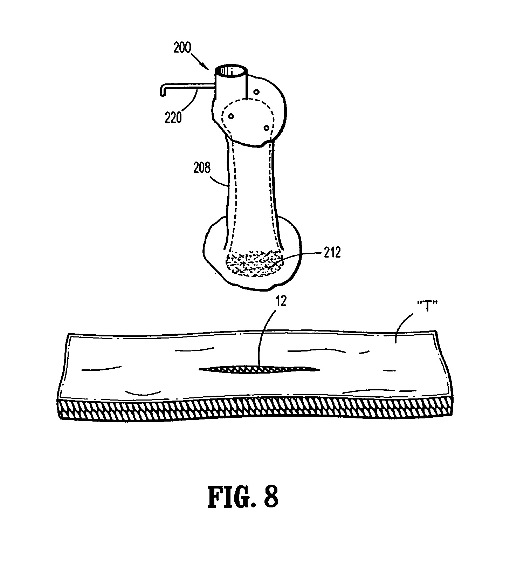

As with seal anchor member **100** discussed above with respect to **FIGS. 1-6****,** seal anchor member **200** is adapted to transition from an expanded condition (**FIG. 7**) to a compressed condition (**FIG. 8**). In the compressed condition (**FIG. 8**), seal anchor member **200** is configured for insertion within tissue tract **12** in tissue **"T",** in a similar manner, as discussed above with respect to seal anchor member **100** (**FIGS. 1-5**). Seal anchor member **200** is positioned within tissue **"T"** whereby foam segment **212** of the seal anchor member **200** and assumes the expanded condition. Fluid port **214** may be connected to fluid source **216** (**FIG. 7**) and fluid is communicated into the internal cavity **210** defined by membrane **208.** As internal cavity **210** fills with fluid, the dimensions of internal cavity **210** and membrane **208** are enlarged, thereby forcing the outer surface of seal anchor member **200** outwardly and establishing a seal within the incision **"I".**

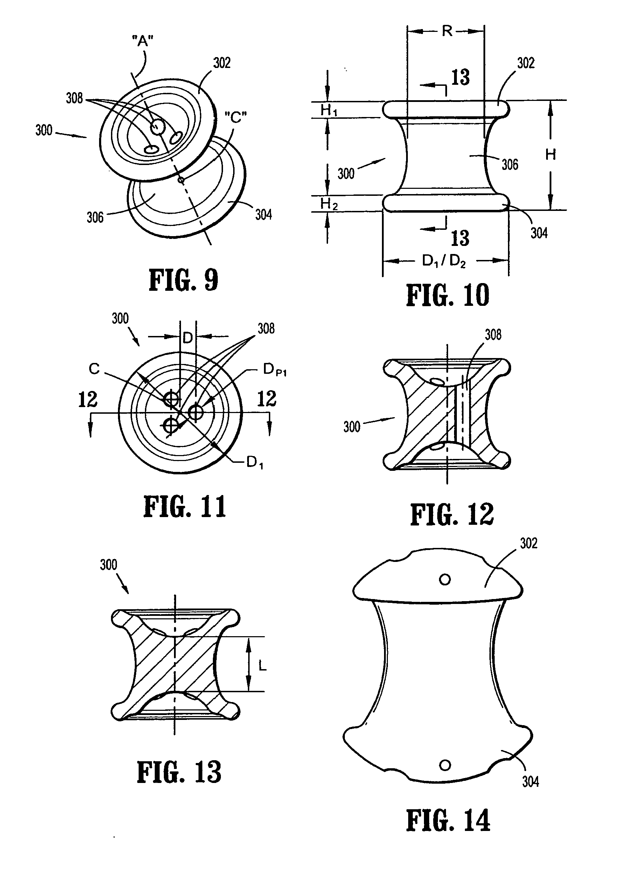

With reference now to **FIGS. 9-13****,** another embodiment of a seal anchor member 300 is disclosed. Seal anchor member **300** extends along a longitudinal axis **"A"** that passes through a centerpoint **"C"** thereof. Seal anchor member **300** defines an overall axial dimension **"H"** measured along the longitudinal axis **"A".** The overall axial dimension **"H"** will generally lay substantially within the range of approximately **25**mm to approximately **75**mm, and desirably, is approximately equal to **50**mm. However, the present disclosure also contemplates a seal anchor member **300** that defines either a substantially larger or smaller overall axial dimension **"H".**

As with each of the previous embodiments, the material comprising seal anchor member **300** is sufficiently compliant to accommodate off-axis movement of the surgical object, or objects, **"I"** inserted therethrough that may be necessitated during the course of the minimally invasive surgical procedure in which seal anchor member **300** is employed. In one embodiment, seal anchor member **300** is formed from a suitable foam material, which may be at least partially constituted of polyisoprene, urethane, or silicone, or the like. Alternatively, seal anchor member **300** may be formed of a biocompatible gel material.

As with the previous embodiments, seal anchor member **300** includes respective trailing (or proximal) and leading (or distal) ends **302, 304,** an intermediate portion **306** disposed therebetween, and one or more ports **308** that extend longitudinally between the respective trailing and leading ends **302, 304** and through seal anchor member **300.**

Proximal end **302** of seal anchor member **300** defines a first radial dimension **D₁** and a first axial dimension **H₁,** and distal end **304** defines a second radial dimension **D₂** and a second axial dimension **H₂.** The present disclosure contemplates a seal anchor member **300** having proximal and distal ends **302, 304** that define radial dimensions **D₁. D₂** generally laying substantially within the range of approximately **25**mm to approximately **75**mm, and axial dimensions **H₁, H₂** generally laying substantially within the range of approximately **6**mm to approximately **11**mm, respectively. Desirably, however, seal anchor member **300** includes proximal and distal ends **302, 304** having radial dimensions **D₁, D₂** that are approximately equal to **50**mm and axial dimensions **H₁, H₂** that are approximately equal to **8.5**mm, respectively. A seal anchor member **300** having proximal and distal ends **102, 104** that define substantially larger or smaller radial and axial dimensions is also within the scope of the present disclosure.

In the embodiment illustrated in **FIGS. 9-13****,** seal anchor member **300** includes respective proximal and distal ends **302, 304** having respective first and second radial dimensions **D₁, D₂** that are substantially equivalent. However, an embodiment of seal anchor member **300** that includes respective proximal and distal ends **302, 304** having respective first and second radial dimensions **D₁, D₂** that differ is also contemplated herein.

Intermediate portion **306** of seal member **300** defines a radial dimensions **"R"** generally laying substantially within the range of approximately **20**mm to approximately **50**mm, and an axial dimension **"L"** generally laying substantially within the range of approximately **10**mm to approximately **40**mm. While it is desirable for the radial and axial dimensions **"R", "L"** of intermediate portion **306** to be approximately equal to **35**mm and **25**mm, respectively, a seal anchor member **300** having an intermediate portion **306** that defines substantially larger or smaller radial and axial dimensions is not beyond the scope of the present disclosure. The radial dimension **"R"** of intermediate portion **306** may be substantially uniform or variable along the axial dimension **"L"** thereof, and may be appreciably less than, greater than, or equal to the respective radial dimensions **D₁, D₂** of proximal and distal ends **302, 304,** as discussed above.

As with each of the previous embodiments, the port, or ports, **308** are configured to removably receive a surgical object **"I"** (not show), and prior to the insertion of surgical object **"I",** each port **308** defines an initial dimension **D_{P1}. D_{P1}** will generally lie substantially within the range of approximately **0**mm to approximately **13**mm, and desirably, is approximately equal to **6.5**mm. However, a seal anchor member **300** having a port **308** that defines a substantially greater initial dimension **D_{P1}** is not beyond the scope of the present disclosure. In those embodiments of seal member **300** employing a port **308** that defines an initial dimension **D_{P1}** approximately equal to **0**mm the escape of insufflation gas (not shown) therethrough may be substantially prevented in the absence of surgical object **"I".**

Seal anchor member **300** may include a plurality of ports **308** that are symmetrically arranged with respect to the longitudinal axis **"A".** It is further contemplated that each port **308** may be spaced equidistant from the longitudinal axis **"A".** In one embodiment, each port **308** is spaced a distance **"D"** from the longitudinal axis **"A"** generally laying substantially within the range of approximately **6**mm to approximately **11**mm, and desirably, approximately equal to **8.5**mm. However, in alternate embodiments, seal anchor member **300** may include ports **308** spaced either a larger or smaller distance from the longitudinal axis **"A".** Ports **308** may be arranged such that they are spaced equally from one another, or alternatively, the distance between adjacent ports **308** may vary.

Either or both of the respective proximal and distal ends **302, 304** of seal anchor member **300** define surfaces that are substantially arcuate, e.g., concave, as seen in **FIGS. 9-13****,** to facilitate insertion of seal anchor member **300** within a tissue tract **12** (**FIG. 1**) defined by tissue surfaces **14** and formed in tissue **"T",** e.g., an incision, as discussed above. The concave orientation may, e.g., assist in guiding a surgical instrument toward one of ports **308** and also confine the tip of the instrument within the outer boundary of the proximal end **302** of seal anchor member **300.** In the alternative, either or both of proximal and distal ends **302, 304** may be convex as seen in **FIG. 14****.**

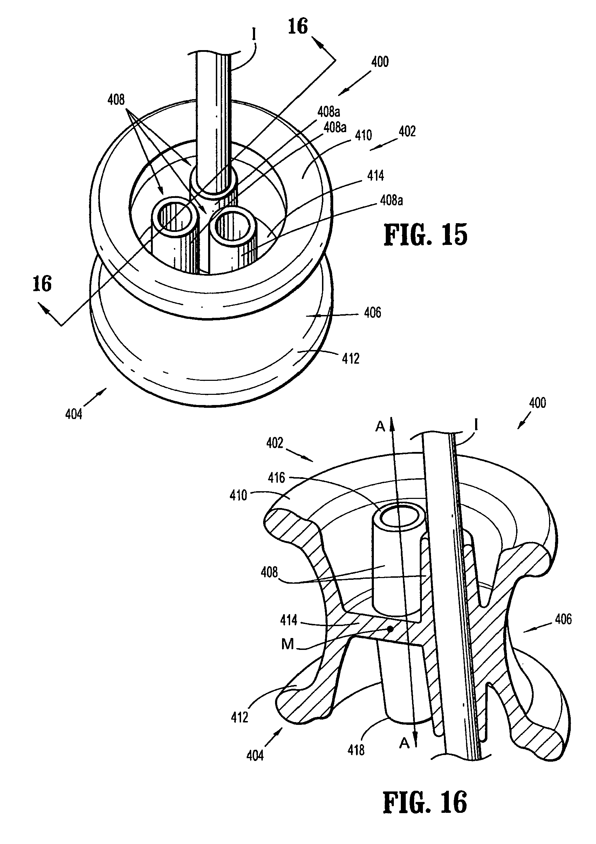

Referring now to **FIGS. 15-16****,** another embodiment of seal anchor member **400** is disclosed. Seal anchor member **400** includes respective proximal and distal ends **402, 404,** an intermediate portion **406** disposed between the proximal and distal ends **402, 404,** and one or more generally tubular port segments **408** defining ports **408a** that extend longitudinally through seal anchor member **400** and between the proximal and distal ends **402, 404.** The seal anchor member **400** is substantially similar to the seal anchor **100** illustrated in **FIGS. 1-5****,** and accordingly, will only be discussed with respect to its differences.

In one embodiment, as seen in **FIGS. 15-16****,** seal anchor member **400** defines corresponding proximal and distal rims **410, 412,** respectively. The proximal and distal rims **410, 412** facilitate deformation of seal anchor member **400** from the expanded condition (**FIGS. 15-16****)** to the compressed condition (not shown) and the anchoring of seal anchor member **400** within tissue, as previously discussed with respect to the seal anchor member **100** illustrated in **FIGS. 1-5****.**

Tubular port segments **408** are secured to the intermediate portion **406** by a connective member **414** such that the longitudinal position of the port segments **408** remain substantially constant with respect to the respective proximal and distal rims **410, 412** during insertion and removal of the surgical object **"I".** In the embodiment illustrated in **FIGS. 15-16****,** the connective member **414** extends inwardly from the intermediate portion **406** and is attached to ports **408** at midpoints **"M"** thereof that are spaced equidistant from the respective proximal and distal rims **410, 412.** In various embodiments, the connective member **414** may be composed of the same material comprising the seal anchor member **400,** or alternatively, the connective member **414** may be composed of a material that is substantially more rigid, to inhibit off-axis movement of the surgical object **"I"** following its insertion into one of the ports **408,** or substantially less rigid, to facilitate off-axis movement of the surgical object **"I".**

In the embodiment illustrated in **FIGS. 15-16****,** the ports **408** extend longitudinally along the longitudinal axis **"A"** defined by the seal anchor member **400** such that a proximal end **416** of the ports **408** is coplanar with the proximal rim **402** and a distal end **418** of the ports **408** is coplanar with the distal rim **404.** However, embodiments in which the proximal and distal ends **416, 418** of ports **408** extend beyond the proximal and distal rims **402, 404,** respectively, such that they extend at least partially from the intermediate portion **406,** and embodiments in which the proximal and distal ends **416, 418** of ports **408** are defined entirely within the intermediate portion **406** are also contemplated herein.

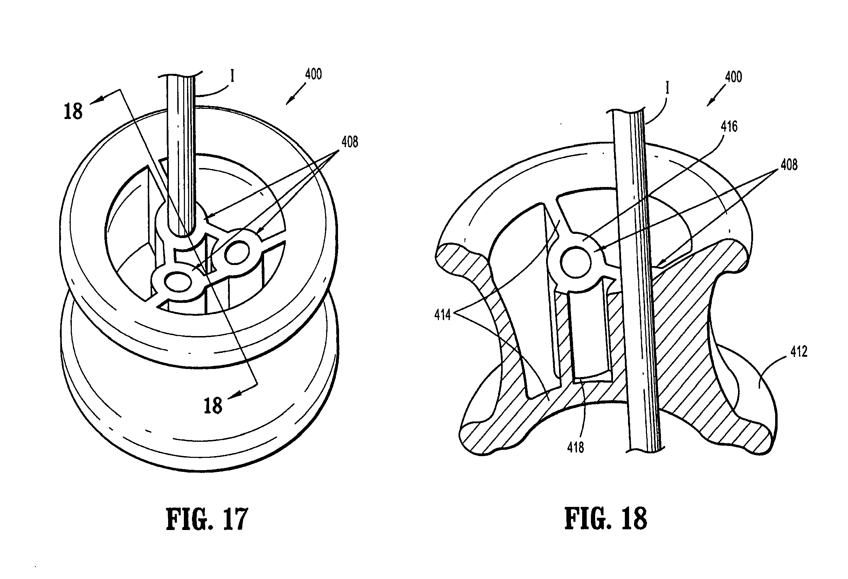

Referring now to **FIGS. 17-18****,** in an alternate embodiment, the connective member **414** extends inwardly from the distal rim **412** and is attached to ports **408** at the distal ends **418** thereof. To further limit off-axis movement of the surgical object "I" upon insertion, the connective member **414** may extend substantially along the length of the ports **408,** as illustrated. Either or both of the respective proximal and distal ends **416, 418** of the ports **408** may be beveled, e.g., to facilitate the insertion and removal of the surgical object **"I".**

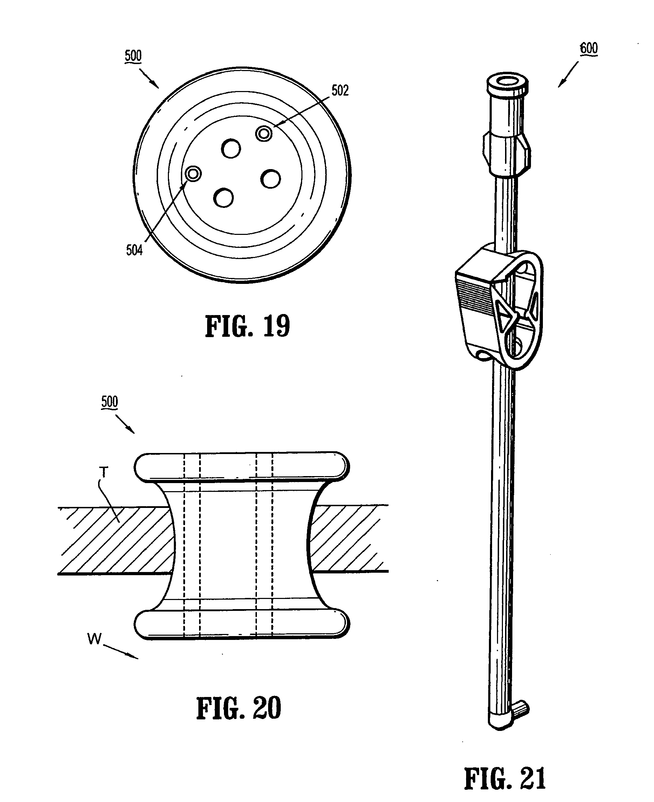

**FIGS. 19-20** illustrate an alternate embodiment of the seal anchor member, referred to generally by reference number **500.** The seal anchor member **500** is substantially similar to the seal anchor member **300** discussed above with respect to **FIGS. 9-14****,** and accordingly, will only be discussed with respect to its differences therefrom.

The seal anchor member **500** includes an ingress port **502** and an egress port **504** extending longitudinally through the seal anchor member **500.** The ingress port **502** facilitates the communication of a fluid through the seal anchor member **500** and into a surgical worksite **"W"** located beneath the patient's tissue **"T".** In one embodiment, the ingress port **502** is configured and dimensioned to removably receive a tube assembly **600** (**FIG. 21**) to facilitate insufflation of the surgical worksite **"W".** In contrast, the egress port **504** facilitates the communication of a fluid, such as smoke, through the seal anchor member **500** and out of the surgical worksite **"W".** To substantially limit the communication of fluid into and out of the surgical worksite **"W",** the ingress and egress ports **502, 504** may respectively include a one-way valve (not shown), such as a duck-bill or zero closure valve. Alternatively, the ingress port **502** and the egress port **504** may be normally biased towards a closed condition.

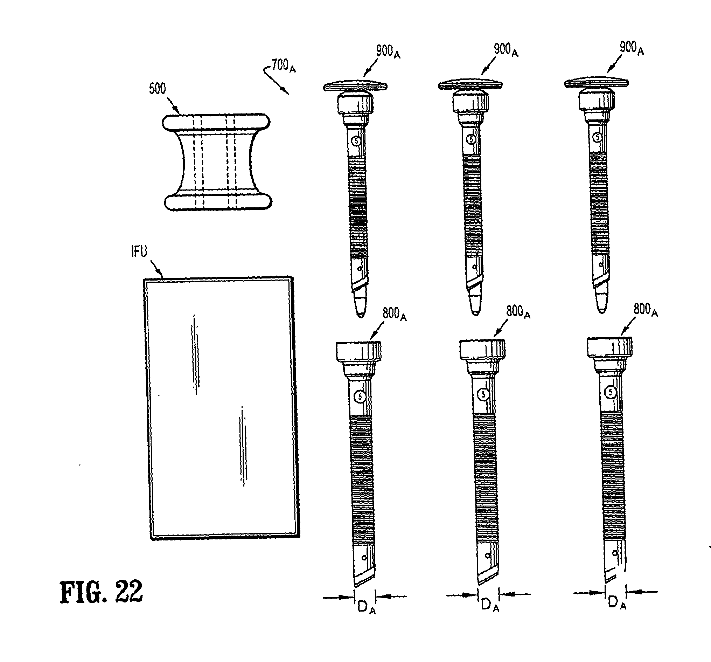

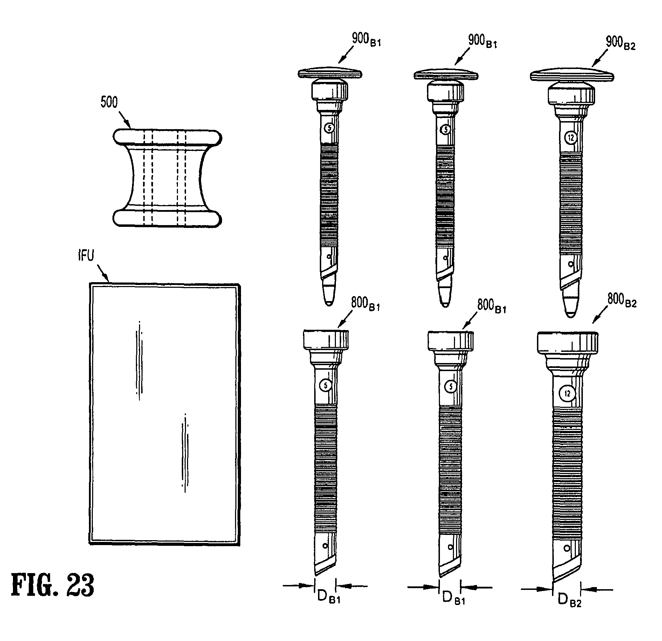

With reference now to **FIGS. 22-23****,** kits according to the present disclosure include a seal anchor member, one or more cannulae, and one or more obturators together with instructions for use "IFU". In one embodiment, a first kit **700_{A}** is disclosed that includes the seal anchor member **500** discussed above with respect to **FIGS. 19-20****,** three cannulae **800_{A}** each defining an outer diameter **"D_{A}"** of **5**mm, and three obturators **900_{A}** configured for removable insertion through the cannulae **800_{A}.** In another embodiment, a second kit **700_{B}** is disclosed that includes the seal anchor member **500** discussed above with respect to **FIGS. 22-23****,** two cannulae **800_{B1}** each defining an outer diameter **"D_{B1}"** of **5**mm, two obturators **900_{B1}** configured for removable insertion through the cannulae **800_{B1},** a single cannula **800_{B2}** defining an outer diameter **"D_{B2}"** of **12**mm, and a single obturator **900_{B2}** configured for removable insertion through the cannulae **800_{B2}.**

The kit components will typically be maintained within sterile packaging, with individual components being packaged either together or separately in different sterile containers. Usually, even when packaged in separate sterile containers, all components of the kit will be placed together within a common package. The instructions for use "IFU" may be provided on a separate printed sheet, such as a conventional package insert, or may be printed in whole or in part on other portions of the packaging or the device itself.

While the kits **700_{A}, 700_{B}** have been described as including the seal anchor member **500** and three cannulae with corresponding obturators of specific dimensions, it should be understood that kits according to the present disclosure may alternatively include any of the seal anchor members described herein above in combination with any desired number of cannulae and obturators exhibiting any suitable dimensions.

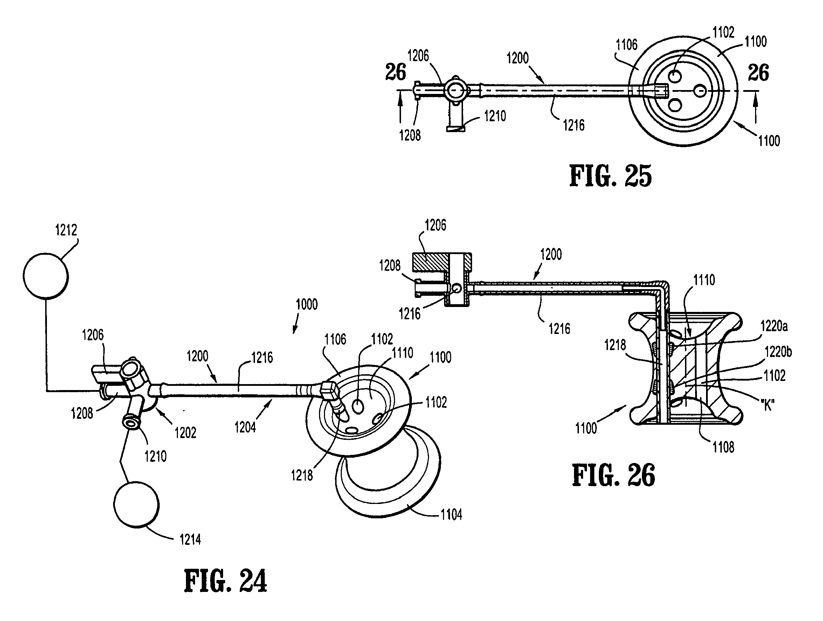

**FIGS. 24-26** illustrate another embodiment of the surgical kit. Surgical kit **1000** includes seal anchor member **1100** and fluid delivery, e.g., insufflation/evacuation instrument, **1200** which is positionable within the seal anchor member **1100.** Seal anchor member **1100** includes a plurality of passageways **1102** (e.g., four are shown).extending through the seal anchor member **1100,** Passageways **1102** may extend in general parallel relation with respect to the longitudinal axis **"k".** In the alternative, passageways **1102** may be in oblique relation with respect to the longitudinal axis **"k"** to provide specific directional capability to the seal anchor member **1100,** whereby an instrument may be advanced at a predetermined angular orientation relative to the longitudinal axis **"k".** Passageways **1102** may be radially spaced about the seal anchor member **1100** relative to the longitudinal axis **"k".** In one aspect, passageways **1102** are spaced a predetermined distance sufficient to correspondingly space the instruments introduced within seal anchor member **1100.** This spacing may substantially minimize the potential of engagement of the inserted instruments and enhance freedom of movement above the operative area. Passageways **1102** may be longitudinal bores defined within seal anchor member **1100.** Longitudinal bores may be open in an initial or at rest condition. In the alternative, passageways **1102** may define slits or individual valves, e.g. zero closure valves, which are closed in the normal condition in the absence of an object inserted therethrough. In this embodiment, passageways **1102** would open to permit passage of the surgical object. In either case, upon introduction of the surgical object or instrument, the interior surfaces defining passageways **1102** establish a substantial fluid tight seal about the object.

Seal anchor **1100** defines a substantially hourglass configuration and incorporates enlarged leading and trailing flange segments **1104, 1106** to assist in retention within the body cavity. Leading and trailing end faces **1108, 1110** may be recessed as shown and/or may include any number or shape so as to provide improved compressibility of seal anchor **1100** or freedom of movement of any instruments inserted therethrough. Seal anchor **1100** may be fabricated from any of the aforementioned materials including foam, gel or the like.

Insufflation/evacuation instrument **1200** is adapted for positioning within at least one of the passageways **1102.** Insufflation/evacuation instrument **1200** may be any suitable instrument adapted to convey fluids or introduce insufflation gases, e.g., CO2 into the peritoneal cavity, and/or evacuate smoke from the cavity. In the depicted embodiment, insufflation instrument **1200** includes housing **1202** and elongated member **1204** extending from the housing **1202.** Housing **1202** may be fabricated from any suitable material and incorporates a stop cock valve **1206** to permit selective passage and interruption of fluids, e.g., insufflation gases or smoke therethrough. Housing **1202** includes first and second ports or luer connectors **1208,1210** adjacent stop cock valve **1204.** First luer connector **1208** may be adapted for connection to an insufflation source **1212** such as CO2 utilized to insufflate the peritoneal cavity. Second luer connector **1210** may be adapted for fluid connection to an aspiration or gas (e.g. smoke) evacuator **1214.** Stop cock valve **1206** may define opening **1216** which is aligned with either port or luer connector **1208, 1210** through selective rotation of the stop cock valve **1206** thereby selectively fluidly connecting the insufflation source **1212** or the evacuator **1214.** First and second luer connectors **1208, 1210** may be arranged about axes which are substantially perpendicular to each other. Other orientations are also envisioned.

Elongate member **1204** includes first elongate segment **1216** connected to housing **1202** and second elongate segment **1218** extending contiguously from the first elongate segment **1216.** First and second elongate segments **1216, 1218** may be in general alignment with each other. In the alternative, first and second elongate segments **1216, 1218** may be angulated relative to each other at a predetermined angle. In one embodiment, first and second elongate segments **1216, 1218** are arranged at a substantial right angle or perpendicular with respect to each other. This arrangement may facilitate the displacement of housing **1202** and first elongate segment **1216** from the operative area thereby reducing the overall profile of seal anchor member **1100** and insufflation/evacuator instrument **1200.** Elongate member **1204** defines a fluid conduit extending through first and second elongate segments **1216, 1218** and in communication with stop cock valve **1206.** First and second elongate segments **1216, 1218** may be releasably mounted to each other.

Insufflation/evacuator instrument **1200** may be a separate instrument positionable within one of passageways **1102.** In the alternative, seal anchor member **1100** and insufflation/evacuator instrument **1100** may be pre-assembled whereby the insufflation/evacuator instrument **1100** may be permanently connected to the seal anchor member **1100.** In one embodiment, second elongate segment **1218** of insufflation/evacuator instrument **1200** includes external anchors **1220a, 1220b** mounted about the periphery of the second elongate segment **1218.** Anchors **1220a, 1220b** may facilitate retention of second elongate segment **1218** of insufflation/evacuation instrument **1200** within seal anchor member **1110.** Anchors **1220a, 1220b** may be generally annular in configuration or may consist of individual prongs depending outwardly from second elongate segment **1218.** Anchors **1220a, 1220b** are dimensioned to be embedded within the inner surfaces defining the passageway **1102** accommodating insufflation/evacuation instrument. Trailing anchor **1220a** may define an enlarged dimension adjacent its proximal end to resist pull out or retropulsion of insufflation/evacuator instrument **1200.** Leading anchor **1220b** may define an enlarged dimension adjacent its distal end to prevent over insertion of insufflation/evacuator instrument **1200.**

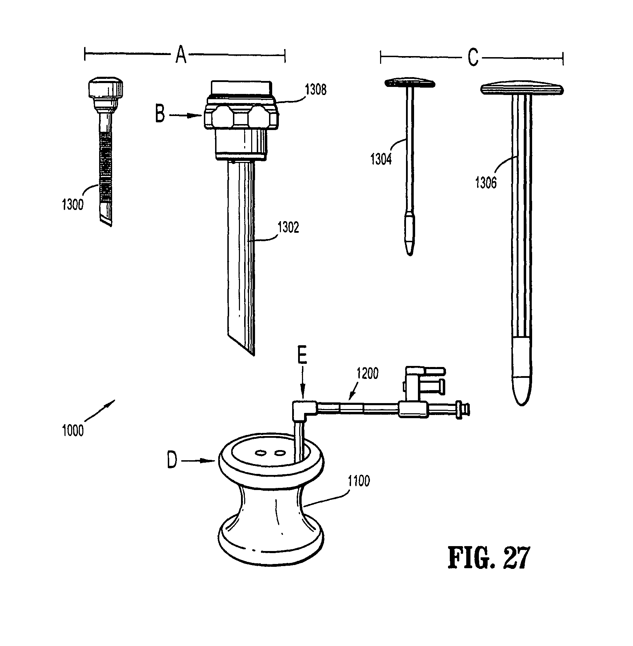

Referring now to **FIG. 27****,** additional instrumentation which may be incorporated within surgical kit **1000** is illustrated. Surgical kit **1000** may further include first and second cannulas **1300, 1302** and first and second obturators **1304, 1306** for respective use with the first and second cannulas **1300, 1302.** First cannula **1300** may be a **5**mm cannula adapted for reception of instrumentation no greater than **5**mm in diameter. First obturator **1304** is positionable within first cannula **1300** to facilitate advancement of the first cannula **1300** through one of passageways **1102** of seal anchor **1100.** Second cannula **1302** may be a **12**mm cannula adapted for reception of instrumentation no greater than **12**mm in diameter and is advanced within seal anchor **1100** with the use of comparably dimensioned second obturator **1306.** Second anchor may incorporate a sealing mechanism such as the sealing system disclosed in commonly assigned U.S. Patent Publication No. 2007/0197972 to Racenet, the entire contents of which are hereby incorporated herein by reference. Surgical kit **1000** may incorporate three or more cannulas with corresponding obturators. Any combinations of sizes of cannulas and obturators are envisioned.

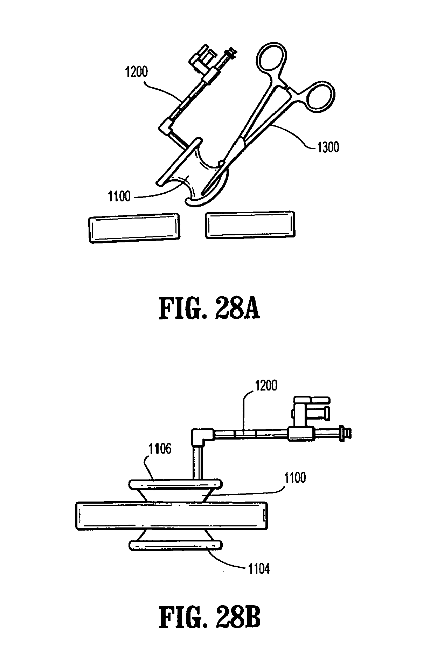

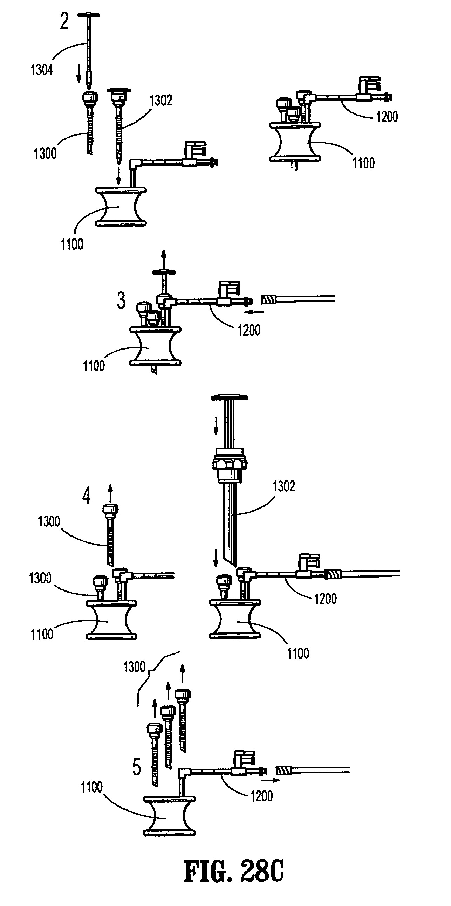

**FIGS. 28A-28C** disclose a method of use of surgical kit. An incision is made in the tissue, e.g., the abdominal tissue, and blunt dissection through the facia and peritoneum is achieved through known methods. Leading flange and end face **1104, 1108** of seal anchor **1100** are manipulated within the incision (**FIG. 28A**), possibly, with the assistance of a surgical clamp **1400.** When appropriately positioned within incision, seal anchor **1100** snugly engages the interior surfaces of the incision with leading and trailing flanges **1104, 1106** adjacent the abdominal lining and outer dermal tissue, respectively (**FIG. 28B**). Thereafter, any combinations of cannulas **1300, 1302** may be introduced within passageways **1102** of seal anchor **1100** with the use of corresponding obturators **1304, 1306.** (**FIG. 28C**) Upon positioning, the obturators are removed thereby providing access through the appropriate cannula **1300, 1302** for passage of surgical instrumentation to perform the surgical procedure. Cannulas **1300, 1302** may be staggered relative to seal anchor **1100** to facilitate freedom of movement above the operative area. Removal of one cannula **1300, 1302** and replacement with another sized cannula **1300, 1302** may be readily achieved. In the event, passageways **1102** of seal anchor **1100** are open in the initial condition (e.g., in the absence of an instrument), the surgeon may place a finger over the passageway upon removal of the cannula and remove the finger when introducing the second cannula within the passageway. Insufflation and/ or evacuation may be continuously effected throughout the procedure with the use of stock cock valve **1204.**

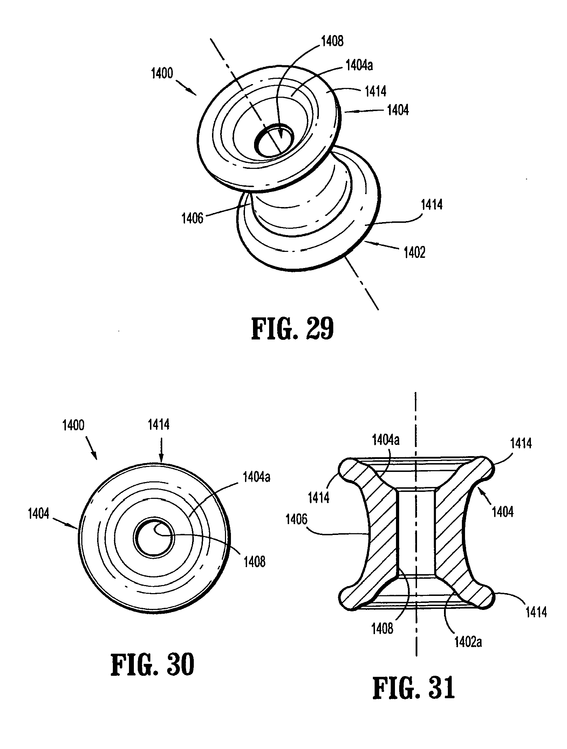

**FIGS. 29-31** illustrate yet another embodiment in which a flexible access device is referred to generally by reference number **1400.**

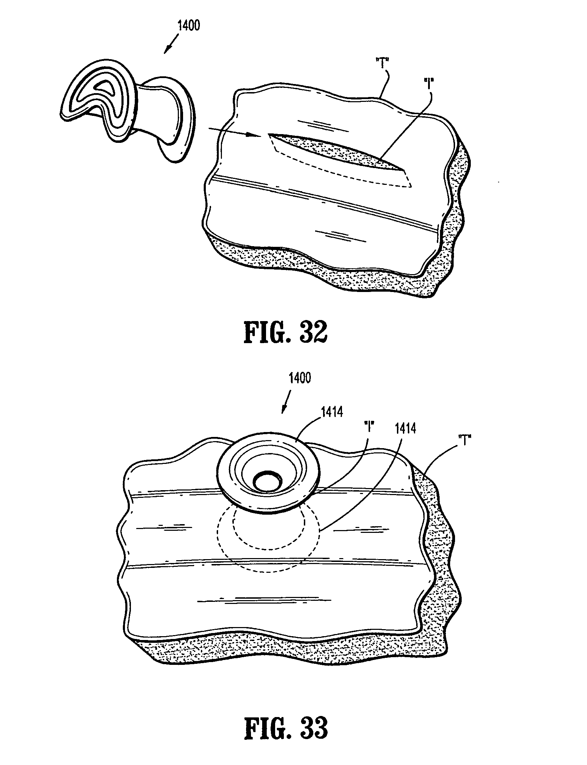

Flexible access device **1400** defines a substantially hourglass shape when viewed from the side and includes respective trailing (or proximal) and leading (or distal) ends **1402, 1404,** respectively, an intermediate portion **1406** disposed between trailing and leading ends **1402, 1404,** and single lumen **1408** that extends longitudinally between the respective trailing and leading ends **1402, 1404** and through intermediate portion **1406.** Positioning member **1414** may be associated with either or both of trailing and leading ends **1402, 1404.** Positioning members **1414** are configured to prevent longitudinal migration of flexible access device **1400** when received through incision **"I"** (**FIG. 32**). As shown, positioning members **1414** are substantially similar in size and/or shape. It is envisioned, however, that position members **1414** may be of different sizes and/or shapes.

Still referring to **FIGS. 29-31****,** intermediate portion **1406** is of a length sufficient that trailing end **1402** is maintained external of the body while leading end **1414** is received within the abdominal cavity. Either or both, trailing (proximal) and leading (distal) ends **1402, 1404** may define concave or tapered receiving and exiting recesses **1402a, 1404a,** respectively. Recesses **1402a, 1404a** are configured to facilitate insertion of an instrument therethrough. The flexible nature of flexible access device **1400** permits instruments having irregular shapes, such as non-linear or curved profiles to be received therethrough. When flexible access device **1400** is used in a procedure requiring insufflation of the body cavity, flexible access device **1400** is configured to form a seal with tissue **"T"** around incision "I" and the instrument inserted therethrough. Alternatively, an access cannula (not shown), may be inserted through port **1408.** The access cannula may or may not include a seal.

Flexible access device **1400** may be formed of materials similar to those for the seal anchor member, such as, for example, silicone, thermoplastic elastomers (TPE), rubber, foam gel, etc. Flexible access device **1400** is formed as a single body that is compressible in both radial and longitudinal dimensions. In this manner, flexible access device **1400** may be compressed or squeezed and inserted through an incision in the body of a patient. In one embodiment, flexible access device **1400** includes TPE material that is infused with an inert gas, e.g. CO₂ or Nitrogen, to form a foam structure. Flexible access device **1400** may be coated with a lubricant, e.g. Parylene N or C, in order to create a lubricious surface finish on all external surfaces. Various other coatings, e.g., hydrophilic, hydrophobic, bio-agents, anti-infection, analgesic, may also be employed to modify the properties of flexible access device **1400.** The coating may facilitate insertion of flexible access device **1400** into an incision and insertion of instruments therethrough.

Lumen **1408** extends through flexible access device **1400** and defines longitudinal axis configured to receive surgical instrument in a sealing manner. Lumen **1408** may include a protective coating or sleeve (not shown), extending the length of flexible access device **1400** to prevent tearing of flexible access device **1400** during insertion and removal of surgical instruments. The sleeve or coating may also facilitate insertion and removal of surgical instruments **50.** The sleeve may be integrally formed with flexible access device **1400,** or instead may be securely affixed to flexible access device **1400** using adhesive, ultrasonic welding or other suitable means.

Referring now to **FIGS. 32-34****,** the use of flexible access device **1400** in a single incision surgical procedure will now be described. Although flexible access device **1400** will be described as relates to relates to a procedure for resectioning a body organ, the aspects of the present disclosure may be modified for use in a variety of procedures and should not be read as limited to the procedure herein described.

Referring initially to **FIG. 32****,** once incision **"I"** has been formed through body tissue **"T",** flexible access device **1400** is squeezed or compressed to reduce flexible access device **1400** to a relatively smaller diameter for insertion through incision **"I".** As noted hereinabove, flexible access device **1400** is formed of a flexible material which allows flexible access device **1400** to be compressed. It should be recognized that flexible access device **1400** may be compressed into any suitable configuration prior to being inserted into an incision, not merely the configuration shown in **FIG. 32****.** For example, in one embodiment, prior to insertion flexible access device **1400** is clamped at leading end **1402** while trailing end **1404** remains essentially uncompressed, and clamped trailing end **1404** is inserted into incision **"I".** In another embodiment, an insertion mechanism (not shown) is used to insert flexible access device **1400** into incision **"I".**

Referring to **FIG. 33****,** once flexible access device **1400** has been inserted through incision **"I",** pressure on flexible access device **1400** is released, allowing flexible access device **1400** to return towards its initial uncompressed state within incision **"I".** Typically, incision **"I"** is formed having a size that is smaller than the diameter of the initial uncompressed state of flexible access device **1400.** In this manner, when in place within the incision **"I",** flexible access device **1400** contacts and presses against the inner surface of incision **"I",** thereby retracting the opening and sealing with incision **"I".** Since incisions are often slit-shaped when formed, the portion of flexible access device **1400** that is located within incision **"I"** may be somewhat oval-shaped (when viewed from above). As noted hereinabove, flexible access device **1400** includes positioning members **1414** to prevent migration of flexible access device **1400** through incision **"I".**

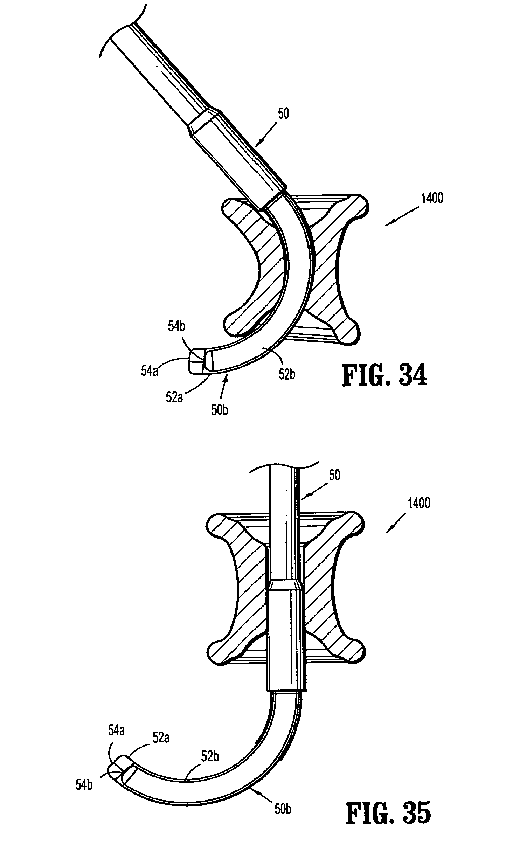

Turning to **FIGS. 34** and **35****,** once flexible access device **1400** has been positioned above a target site, a surgical instrument having an irregular profile, e.g., surgical stapler **50,** may be directly inserted through lumen **1408** to operate at the surgical site. Surgical stapler **50** includes curved first and second jaws **52a, 52b** each having a free end **54a, 54b,** respectively. As shown, curved first jaw **52a** includes a surgical stapling cartridge. It is envisioned that surgical instrument **50** may be received through flexible access device **1400** prior to insertion of flexible access device **1400** through incision **"I".** The body cavity may or may not be insufflated, depending on the procedure being performed. It is envisioned that the insufflation gas may be provided to the body cavity through an instrument inserted through lumen **1408,** or instead, through an alternate access device (not shown), e.g., a cannula, trocar and/or other insufflation needle inserted through another incision. Due to the flexible nature of flexible access device **1400,** once instrument **50** is inserted through flexible access device **1400,** a proximal end **50a** of instrument **50** may be manipulated in any direction, as indicated by arrows **"B".** Thus, seal anchor member **1400** permits a surgeon to manipulate or orient instrument **50** at various locations relative to the target site.

Upon completion of the procedure, instrument **50** is removed from lumen **1408** of flexible access device **1400** and flexible access device **1400** is compressed or squeezed such that it may be removed from incision **"I".** It is envisioned that flexible access device **1400** may be removed from incision **"I"** prior to instrument **50** being removed therefrom. In this manner, both instrument **50** and flexible access device **1400** are removed simultaneously. Incision **"I"** is then closed in a conventional manner.
The invention may be described by reference to the following numbered paragraphs:-

A flexible access device according to the present invention having a body and a lumen extending through the body, the lumen for forming a seal with a non-linear instrument disposed in the lumen, for use in a method of accessing an abdominal cavity, wherein the method comprises the steps of:;
creating an incision through the abdominal wall;
compressing the body such that it may be inserted through the incision;
inserting the compressed body through the incision;
releasing the compressed body to permit the body to return towards an original shape; and
receiving a non-linear instrument through the lumen.

The flexible access device of paragraph **[00107],** further including the step of removing the non-linear instrument.

The flexible access device of paragraph **[00107],** wherein the body includes a Parylene coating.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

## Claims

1. A flexible access device for insertion through tissue, comprising:
a compressible body having a first collapsed configuration and a second resiliently expanded configuration, the body being compressible in both a radial dimension and a longitudinal dimension and being resilient to expand in an incision in the tissue, the body having a trailing end defining concave receiving recess and a leading end defining a concave exiting recess; and
a lumen disposed in the body and extending therethrough, the lumen communicating with the concave receiving and exiting recesses so as to receive an instrument with a non-linear shaft.

2. The flexible access device as recited in claim 1, wherein the body has a central portion and the trailing end includes a positioning member.

3. The flexible access device as recited in claim 1 or 2, wherein the leading end of the body includes a positioning member.

4. The flexible access device as recited in claim 3, wherein the positioning member has a diameter greater than a diameter of the central portion.

5. The flexible access device as recited in any preceding claim, wherein the body includes a coating that is at least one of parylene, hydrophilic, hydrophobic, bio-agents, anti-infection and analgesic.

6. A flexible access device according to any preceding claim having a body and a lumen extending through the body, the lumen for forming a seal with a non-linear instrument disposed in the lumen, for use in a method of accessing an abdominal cavity, wherein the method comprises the steps of:;
creating an incision through the abdominal wall;
compressing the body such that it may be inserted through the incision;
inserting the compressed body through the incision;
releasing the compressed body to permit the body to return towards an original shape; and
receiving a non-linear instrument through the lumen.

7. The flexible access device of claim 8, further including the step of removing the non-linear instrument.

8. The flexible access device of claim 8, wherein the body includes a Parylene coating.

9. A kit for performing a lower anterior resection, the kit comprising:
a surgical instrument having a pair of jaws for applying surgical fasteners to tissue, the pair of jaws having free ends and a curved configuration; and
a flexible access device comprising:
a compressible body having a first collapsed configuration and a second resiliently expanded configuration, the body being compressible in both a radial dimension and a longitudinal dimension and being resilient to expand in an incision in the tissue, the body having a trailing end defining concave receiving recess and a leading end defining a concave exiting recess; and
a lumen disposed in the body and extending therethrough, the lumen communicating with the concave receiving and exiting recesses so as to receive an instrument with a non-linear shaft.

10. The kit of claim 9, wherein the surgical instrument includes a surgical stapling cartridge.
